(19) Europäisches Patentamt
European Patent Office
Office européen des brevets

(11) **EP 4 360 537 A1**

(12) **EUROPEAN PATENT APPLICATION**

(43) Date of publication:
**01.05.2024 Bulletin 2024/18**

(21) Application number: **22203205.4**

(22) Date of filing: **24.10.2022**

(51) International Patent Classification (IPC):
***A61B 5/00*** *(2006.01)*

(52) Cooperative Patent Classification (CPC):
**A61B 5/4088;** A61B 5/16; A61B 5/4064

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
- **SPELT, Hanne Adriana Alijda**
  **Eindhoven (NL)**
- **KLAMING, Laura**
  **Eindhoven (NL)**
- **VAN EE, Raymond**
  **5656AG Eindhoven (NL)**

(74) Representative: **Philips Intellectual Property &
Standards
High Tech Campus 52
5656 AG Eindhoven (NL)**

(54) **COGNITIVE ASSESSMENT APPARATUS**

(57)    The present invention relates to cognitive assessment apparatus (10), comprising:
- an input/output unit (20);
- at least one sensor (30); and
- a processing unit (40).

The input/output unit is configured to present a cognitive test to a user. The at least one sensor is configured to acquire at least one bio signal of the user as the user undertakes the cognitive test. The processing unit is configured to determine a cognitive effort index, wherein the determination of the cognitive effort index comprises utilization of the at least one bio signal of the user. The processing unit is configured to determine a cognitive assessment, wherein the determination of the cognitive assessment comprises utilization of the cognitive effort index.

FIG. 2

EP 4 360 537 A1

**Description**

FIELD OF THE INVENTION

The present invention relates to a cognitive assessment apparatus, a cognitive assessment system, and a cognitive assessment method, as well as to a computer program element and a computer readable medium.

BACKGROUND OF THE INVENTION

Traumatic brain injury (TBI) constitutes a large, and rapidly growing public health concern. Each year, around 69 million people worldwide sustain a TBI. After arrival at the hospital, a CT scan is made if the neurologist suspects the presence of a life-threatening condition, in particular haemorrhagic bleedings. If no gross abnormalities are found, patients are sent home with the diagnosis mild Traumatic Brain Injury (mTBI). Most traumatic brain injuries (TBIs) are classified as mild. (>85%).

Traditionally, mild Traumatic Brain Injury (mTBI) is difficult to diagnose; CT scans, which have a low resolution, typically do not detect brain damage, and neuropsychological assessment (NPA) test scores normalize between 1 week and 3 months after injury (see: Karlsen, R. H., Saksvik, S. B., Stenberg, J., Lundervold, A. J., Olsen, A., Rautio, I., ... & Skandsen, T. (2021). Examining the Subacute Effects of Mild Traumatic Brain Injury Using a Traditional and Computerized Neuropsychological Test Battery. Journal of Neurotrauma, 38(1), 74-85). However, mTBI patients may continue to experience cognitive problems in their daily live. Often, mTBI patients (and patients with another neurological condition that can cause subtle cognitive impairment, e.g. MS or stroke) may perform a neuropsychological assessment within the norm.

Around 50% of mTBI patients develop chronic problems., They may exhibit persistent behavioural, emotional, and especially cognitive symptoms. Patients may be referred to specialized rehabilitation clinics where diagnostic cognitive tests are administered and rehabilitation therapy is provided, but no further brain scans are made. Even in specialized rehabilitation clinics, cognitive problems may easily be overlooked.

The gold standard for assessing the degree of cognitive impairment in clinical practice involves paper-and-pencil neuropsychological tests. Such a paper-and-pencil neuropsychological test is the Trail Making Test. Information regarding the Trail Making Test can be found in the following article: Vermeent, S., de Oliveira, M. & Schmand, B. (2020). "Which cognitive processes are involved in the Trail Making Test? Towards a more precise neuropsychological assessment using digital tablet-based technology" and Bowie, C. R., & Harvey, P. D. (2006). Administration and interpretation of the Trail Making Test. Nature protocols, 1(5), 2277-2281. Npgrj_Nprot_390 2277..2281 (researchgate.net).

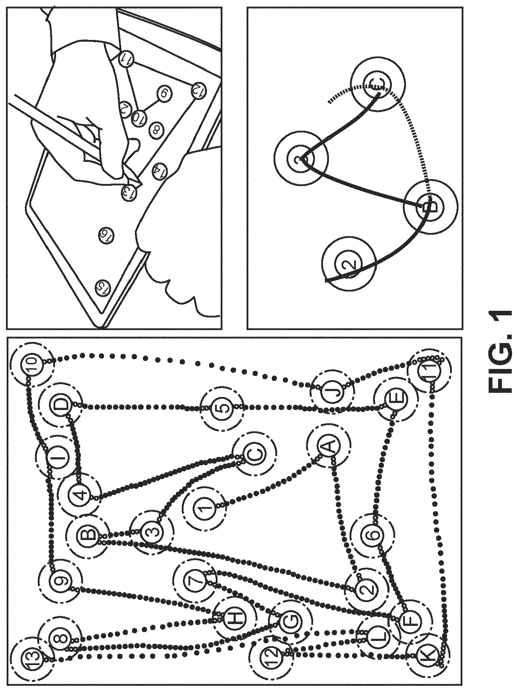

Philips Healthcare has automated the paper and pencil version of the Trail Making Test and made it accessible in digital tablet-based technology. Therefore, it becomes technically possible to track the actual behaviour of a participant continuously in space (x, y coordinates) and in time (time to reach a target, time to dwell on a target, total path time...). These features allow for more precise performance measurement. Fig. 1 shows The Trail Making Test in digital tablet-based technology as implemented by Digital Cognitive Diagnostics, Philips Healthcare.

However, standard (paper-pencil) cognitive tests are not sensitive enough to pick up subtle cognitive impairment. Patients with subtle cognitive deficits, such as mTBI patients, do not show profound abnormalities on these standardized tests despite significant subjective complaints of cognitive dysfunction in daily life. If performance meets norms, patients are considered unimpaired. However, standardized tests are inadequate for diagnosis of subtle cognitive deficits because dependent measures (such as reaction times and error rates) merely reflect the end-result of a diversity of underlying cognitive processes. In other words, test outcomes inform us on whether patients were able to complete a task, not on how they have completed a task.

There is a need to resolve these issues.

SUMMARY OF THE INVENTION

It would be advantageous to provide a technique to assess whether a patient has a cognitive impairment, including a subtle cognitive impairment, such as mTBI.

The object of the present invention is solved with the subject matter of the independent claims, wherein further embodiments are incorporated in the dependent claims. It should be noted that the following described aspects and examples of the invention apply to the cognitive assessment apparatus, the cognitive assessment system, the cognitive assessment methods as well as to the computer program element and the computer readable medium.

In a first aspect, there is provided a cognitive assessment apparatus, comprising:

- an input/output unit;
- at least one sensor; and

- a processing unit.

The input/output unit is configured to present a cognitive test to a user. The at least one sensor is configured to acquire at least one bio signal of the user as the user undertakes the cognitive test. The processing unit is configured to determine a cognitive effort index. The determination of the cognitive effort index comprises utilization of the at least one bio signal of the user. The processing unit is configured to determine a cognitive assessment. The determination of the cognitive assessment comprises utilization of the cognitive effort index.

Thus, cognitive effort during, and indeed after, a neuropsychological assessment is measured using a bio signal and this is analyzed to provide an additional metric for diagnosis. It can then be determined, on the basis of the cognitive assessment, if a patient should undergo a costly scan such as a MRI or PET scan.

In this manner, cognitive effort during the performance of a cognitive test gives an indication about subtle cognitive impairments (e.g., mTBI, MS, Stroke), that are not currently measured in a neuropsychological assessment.

To put this another way, measuring effort in addition to test performance can provide a more sensitive outcome measure for subtle cognitive impairment, where measuring the physiological load during performance of a test can provide additional information on the cognitive effort spent during the test.

In an example, the determination of the cognitive assessment comprises a comparison of the cognitive effort index with a reference cognitive effort index or a comparison with a cognitive effort threshold.

In an example, the input/output unit is configured to present one or more further cognitive tests to the user. The at least one sensor is configured to acquire at least one bio signal of the user as the user undertakes each further cognitive test of the one or more further cognitive tests. The processing unit is configured to determine a further cognitive effort index for each of the one or more further cognitive tests. The determination of the further cognitive effort index comprises utilization of the at least one bio signal of the user for each of the one or more further cognitive tests. The determination of the cognitive assessment for the user comprises utilization of the further cognitive effort index for each of the one or more further cognitive tests.

In an example, the determination of the cognitive assessment for the user comprises a determination of a degree of variability between the cognitive effort index and at least one of the further cognitive effort indexes.

In this manner it can be determined if a patient has a subtle cognitive impairment, and for example should undergo a costly scan such as a MRI or PET scan, on the basis of fluctuations in effort expended when they carry out tests.

In an example, the one or more further cognitive tests are a plurality of further cognitive tests. The determination of the cognitive assessment for the user comprises a calculation of a curve fitted to the cognitive effort index and the further cognitive effort indexes for the plurality of further cognitive tests.

Thus, longitudinal testing can be used within a test itself, where effort is varying, to determine a cognitive assessment and also longitudinal testing can be utilized across different tests, again where effort is varying, to determine a cognitive assessment. This longitudinal testing can involve curve fitting of data to provide quantitative assessments.

In an example, the input/output unit is configured to receive input from the user as they undertake the cognitive test. The processing unit is configured to determine a performance index for the user undertaking the cognitive test comprising utilization of the input from the user. The determination of the cognitive assessment comprises utilization of the performance index.

Thus, the effort a user expends on a test and their performance in that test can be both used in determining an overall cognitive assessment for the user, enabling it to be determined for example if the user has a cognitive impairment, including subtle cognitive impairment.

It is to be noted that while a "subtle cognitive impairment" is included in the term "cognitive impairment", a "subtle cognitive impairment" is medically different from "cognitive impairment". The new technology described here can determine both cognitive impairment and subtle cognitive impairment.

In an example, the input/output unit is configured to receive input from the user as they undertake each further cognitive test of the one or more further cognitive tests; wherein the processing unit is configured to determine a further performance index for the user undertaking each of the one or more further cognitive test comprising utilization of the input from the user. The determination of the cognitive assessment can comprise utilization of the further performance index for each of the one or more further cognitive tests.

In this manner, the effort a user expends on a test and their performance can be assessed and it can be determined if the performance is plateauing as the user completes numerous tests and becomes more used to the tests and at the same time the cognitive effort can be assessed to assess if the person has indeed peaked in performance or has become bored with the tests, for example. By comparing cognitive effort and performance it can be determined if the user has not yet reached a plateau and their results may not be particularly valid for assessment, determined if the user has indeed reached a plateau and their results are valid for assessment, and if a person has given up on a task if both cognitive effort and performance rise and then fall for example.

To put this another way, as a person carries out trials of a test (or tests of a battery of tests) their performance starts from a baseline and gradually improves towards a plateau that is representative of their actual cognitive capability with respect to the test(s), and it can then be determined to select those tests at the plateau that are representative for further analysis by a medical professional or test overseer, and not utilize the test results leading up to the plateau. The cognitive effort expended by the person provides further information, where it can be determined if the person has really reached a plateau, or is struggling and/or shows anomalous variability in effort that can indicate that the person has a cognitive impairment.

Thus, regarding the example task of pressing a button when a red dot is presented on a screen the instructions associated with the test is "press the button as quickly as you can only when you see a red dot" and the test results in this case comprise the times between the red dot being presented and the time when the button was pressed for the multiple times the red dot was presented on the screen. The fluctuation in the plurality of trials of the test results then comprises the fluctuations in the times between the red dot being shown and the patient pressing the button, and the index for the patient can then comprise this fluctuation information.

In an example, the determination of the cognitive assessment for the user comprises a determination of a degree of variability between the performance index and at least one of the further performance indexes.

In an example, the one or more further cognitive tests are the plurality of further cognitive tests. The determination of the cognitive assessment for the user can comprise a calculation of a curve fitted to the performance index and the further performance indexes for the plurality of further cognitive tests.

Thus, it has been established that model can be fit to the performance improvement of a person as they undertake trials of a test (or tests of a battery of tests) as a performance gradually increases to a plateau. This means that the tests that are at the plateau can be selected for further analysis by medical professional, and the curve itself can be utilized to provide information on the cognitive ability of the person such as through timescale value related to how long it takes the person to reach the plateau and the cognitive effort expended by the person during the taking of the tests provides further information on the cognitive ability of the person. It can also be determined that a person who is not reaching a plateau is bored and not concentrating on the test as there are not expending significant cognitive effort or that they are expending significant cognitive effort but still having trouble completing the tests, that can be indicative of cognitive impairment.

In an example, the determination of the cognitive assessment for the user comprises a correlation of the cognitive index with the performance index and the one or more further cognitive indexes with the one or more further performance indexes.

In an example, the determination of the cognitive assessment comprises a comparison of the cognitive effort index with a reference cognitive effort index or a comparison with a cognitive effort threshold.

In an example, the at least one sensor is configured to acquire at least one bio signal of the user after the user has undertaken the cognitive test. The processing unit is configured to determine a delayed cognitive effort index. The determination of the delayed cognitive effort index comprises utilization of the at least one bio signal of the user acquired after the user has undertaken the cognitive test. The determination of the cognitive assessment can comprise utilization of the delayed cognitive effort index.

In this manner fatigue can be assessed after the cognitive test or battery of tests, in effect as a proxy for effort given during the test or battery of tests, that this assessment of fatigue can be utilized along with the assessment during the test(s) to provide an overall more accurate cognitive assessment.

In an example, the at least one bio signal comprises one or more of: cardiovascular activity, heart rate, heart rate variability, heart high/low frequency ratio, systolic blood pressure, diastolic blood pressure, electrodermal activity, skin conductance level, nonspecific skin conductance responses, respiration rate, respiration depth, pupil dilation, salivation, electroencephalogram data.

In a second aspect, there is provided a cognitive assessment method, comprising: presenting by an input/output unit a cognitive test to a user;

    acquiring by at least one sensor at least one bio signal of the user as the user undertakes the cognitive test;

    determining by a processing unit a cognitive effort index, wherein the determining the cognitive effort index comprises utilizing the at least one bio signal of the user; and

    determining by the processing unit a cognitive assessment, wherein the determining the cognitive assessment comprises utilizing the cognitive effort index.

According to another aspect, there is provided a computer program element controlling one or more of the apparatuses as previously described which, if the computer program element is executed by a processor, is adapted to perform the method as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

**BRIEF DESCRIPTION OF THE DRAWINGS**

Exemplary embodiments will be described in the following with reference to the following drawing:

Fig. 1 shows the Trail Making Test in digital tablet-based technology as implemented by Digital Cognitive Diagnostics, Philips Healthcare;
Fig. 2 shows a schematic example of a cognitive assessment apparatus;
Fig. 3 shows a schematic example of a cognitive assessment method; and
Fig. 4 shows an example of a person's performance with respect to taking trials of a test;
Fig. 5 shows an example of a person's performance with respect to taking trials of a test;
Fig. 6 shows an example of a person's performance with respect to taking trials of a test; and
Fig. 7 shows an example of a person's performance with respect to taking trials of a test.

**DETAILED DESCRIPTION OF EMBODIMENTS**

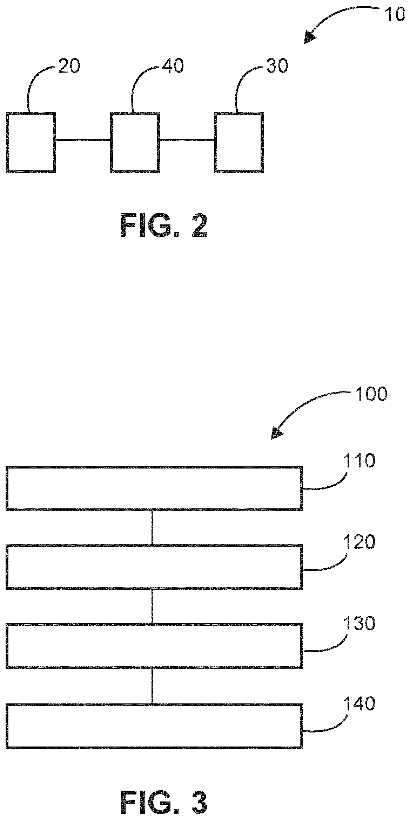

Fig. 2 shows an example of a cognitive assessment apparatus 10 comprising: an input/output unit 20, at least one sensor 30, and a processing unit 40. The input/output unit is configured to present a cognitive test to a user. The at least one sensor is configured to acquire at least one bio signal of the user as the user undertakes the cognitive test. The processing unit is configured to determine a cognitive effort index. The determination of the cognitive effort index comprises utilization of the at least one bio signal of the user. The processing unit is configured to determine a cognitive assessment, wherein the determination of the cognitive assessment comprises utilization of the cognitive effort index.

Thus, cognitive effort during, and indeed after, a neuropsychological assessment is measured using a bio signal and this is analyzed to provide an additional metric for diagnosis.

In this manner, cognitive effort during the performance of a cognitive test gives an indication about subtle cognitive impairments (e.g., mTBI, MS, Stroke), that are not currently measured in a neuropsychological assessment.

To put this another way, measuring effort in addition to test performance can provide a more sensitive outcome measure for subtle cognitive impairment, where measuring the physiological load during performance of a test can provide additional information on the cognitive effort spent during the test.

Here a test means something that the patient is asked to do one time or a number of times over a number of tests - or a battery of tests.

This could for example relate to an instruction for a reaction time task, where a patient is asked to press a button only when they see a red dot on a screen. This can be the test, and the test results can comprise the times between the red dot being shown and the time the patient pressed the button, providing a range in times used in determining a performance index and bio signals of the user can be acquired as they undertake the test to determine a cognitive effort index that is used in determining a cognitive assessment for the user.

The test could for example be a trail making test, where the user is presented with a plurality of targets on an input/output unit and is requested to trace a trail linking targets in a particular order. The test results can be how long the user takes to link the targets, times between targets being linked, number of errors etc.

The test could also for example be to think of as many words as possible starting with the letter E in a 5 minute period. The test result can then be the number of words the user thought of. The user could then be given this test on a periodic basis, for example daily, weekly or monthly, and the variability in the number of words starting with the letter E (the trial results) can be used to determine a performance index for the user and at the same time the bio signals from the patient can be acquired to determine a cognitive index for each test. Also, the user can be provided with a battery of test immediately one after the other, for example thinking of words beginning with E, then a following test to think of words beginning with P, then a following test to think of words beginning with Q. The performance of the user can be determined for each test, and also the bio signals of the user acquired for each test can be used to determine a cognitive effort index for each test that are then used in determining a cognitive assessment for the user.

As the user undertakes the tests, normally the performance of the user is determined to assess the user's cognitive ability. However, it has been determined that by monitoring bio signals of the user a cognitive assessment of the user can be made without having to take into account their performance in the tests. However, taking into account the performance in the tests along with the cognitive effort expended by the user provides for a more accurate determination of cognitive assessment, such as if the person has an impairment and/or helps in selecting test results for further analysis and review.

Thus, user can for example be a patient undergoing some form of psychological test, for example a patient who has had COVID and may be suffering from long COVID, where the impairments are often subtle and difficult to detect through normal cognitive testing, but can be indicated through the newly developed technique. The user can however be someone other than a patient, for example someone undergoing a test for acceptance for further education or a person undergoing a test as part of an application for employment.

In an example, for a trail making test the targets comprise numbered icons.

Details of the Trail Making Test, a well-known validated test, can be found in the following paper: Bowie, C. R., & Harvey, P. D. (2006). Administration and interpretation of the Trail Making Test. Nature protocols, 1(5), 2277-2281. Npgrj_Nprot_390_2277..2281 researchgate.net).

In an example, for a trail making test the specific order of the targets comprises a numbered order of the icons.

In an example, for a trail making test the targets comprise lettered icons.

In an example, for a trail making test the specific order of the targets comprises an alphabetic order of the icons.

In an example, for a trail making test the targets comprise numbered icons and lettered icons.

In an example, for a trail making test the specific order of the targets comprises a numbered order of the numbered icons and an alphabetic order of the lettered icons.

In an example, for a trail making test the specific order of the targets comprises a numbered order of the numbered icons alternating with an alphabetic order of the lettered icons.

In an example, the cognitive test comprises a plurality of tasks (for example linking one target to the next target of a trail making test) and the at least one bio signal is acquired for each task, and the processing unit is configured to determine a cognitive effort index for each task comprising utilization of the associated at least one bio signal acquired for each task, and the determination of the cognitive effort index for the overall test comprises utilization of the cognitive effort index for each task.

In an example, the determination of the cognitive effort index for the overall test comprises a calculation of a curve fitted to the cognitive effort index for each task.

Thus, longitudinal testing can be utilized within a test, as the user expends different levels of effort, to determine an overall cognitive effort index used in determining a cognitive assessment of the user.

According to an example, the determination of the cognitive assessment comprises a comparison of the cognitive effort index with a reference cognitive effort index or a comparison with a cognitive effort threshold.

In an example, the cognitive effort threshold is a pre-set threshold, for example a normalized average or equivalent.

In an example, the cognitive effort threshold is a relative threshold, for example relative to a baseline measurement).

According to an example, the input/output unit is configured to present one or more further cognitive tests to the user. The at least one sensor is configured to acquire at least one bio signal of the user as the user undertakes each further cognitive test of the one or more further cognitive tests. The processing unit is configured to determine a further cognitive effort index for each of the one or more further cognitive tests. The determination of the further cognitive effort index comprises utilization of the at least one bio signal of the user for each of the one or more further cognitive tests. The determination of the cognitive assessment for the user comprises utilization of the further cognitive effort index for each of the one or more further cognitive tests.

In an example, the determination of the cognitive assessment comprises a determination of a standard deviation associated with the cognitive effort index and the further cognitive effort indexes.

Thus, for example regarding the example of the patient being asked to press a button when a dot is shown on a screen and a reaction time between the dot being shown and the button is pressed are tests of a battery of tests, there could be for example 400 tests. This the enables variations/standard deviation of the patient's effort across the tests to be determined.

Thus, for example regarding the example of a patient being asked to think of as many words beginning with E, and then P, and then Q, the effort the user is expending determining from bio signals can be determined and the variation in effort can be determined from which a cognitive assessment of the user can be determined.

In this manner, the presence of subtle cognitive impairment can be detected because a person with a cognitive impairment are generally expected to expend an increased variability in cognitive effort across different tests that exhibit by a person who does not have a cognitive impairment.

It is to be noted that a cognitive effort threshold and cognitive effort indexes across cognitive tests, that enable variability to be assessed, may not generally be used together. Thus, someone may score below the physiological threshold on a multitude of tests within a test battery but does have a very large variability in physiological arousal (does not have a large variability in cognitive effort indexes across cognitive tests) while completing those tests. This large variability then may indicate subtle cognitive impairment even though the thresholds are not reached in any of the tests. Thus, the analysis of multiple tests with respect to variability, without considering thresholds for a test or test, can indicate subtle cognitive impairment, whilst consideration of thresholds without analysing variability may not indicate that there is a subtle cognitive impairment. Thus, for variability in effort between different tests in a test battery, thresholds are not needed, and an average and standard deviation for example would be enough. However, consideration of thresholds can provide further information for the medical professional along with an analysis of variability, for example enabling threshold values to be adjusted and also provide further understanding on the subtle cognitive impairment itself.

According to an example, the determination of the cognitive assessment for the user comprises a determination of a degree of variability between the cognitive effort index and at least one of the further cognitive effort indexes.

According to an example, the one or more further cognitive tests are a plurality of further cognitive tests, and wherein the determination of the cognitive assessment for the user comprises a calculation of a curve fitted to the cognitive effort index and the further cognitive effort indexes for the plurality of further cognitive tests.

Thus longitudinal testing can be used within a test itself, where effort is varying, to determine a cognitive assessment and also longitudinal testing can be utilized across different tests, again where effort is varying, to determine a cognitive assessment. This longitudinal testing can involve curve fitting of data to provide quantitative assessments.

According to an example, the input/output unit is configured to receive input from the user as they undertake the cognitive test. The processing unit is configured to determine a performance index for the user undertaking the cognitive test comprising utilization of the input from the user. The determination of the cognitive assessment comprises utilization of the performance index.

Thus, the effort a user expends on a test and their performance in that test can be both used in determining an overall cognitive assessment for the user, enabling it to be determined for example if the user has a cognitive impairment, including subtle cognitive impairment.

It is to be noted that while a "subtle cognitive impairment" is included in the term "cognitive impairment", a "subtle cognitive impairment" is medically different from "cognitive impairment". The new technology described here can determine both cognitive impairment and subtle cognitive impairment.

The following provides details on different impairments.

**Cognitive impairment** (secondary to brain damage) is commonly classified according to the domain affected. The separation of impairments related to the different sensory modalities is clear: visuoperceptual, visuospatial, and visuoconstructive disorders; disorders of the somatosensory system; and impairments in the auditory system. There are also separate modules for language, memory, attention, and other functions that are superordinate to the sensory or perceptual domains. (Stuss, D.T., Cognitive Impairment, Encyclopedia of the Neurological Sciences (2003), 737-740).

**Subtle cognitive impairment** is defined by a cognitive transitional status involving pathological changes but not sufficiently severe to manifest conspicuous functional loss (Jack CR Jr, Bennett DA, Blennow K, et al. NIA-AA research framework: toward a biological definition of Alzheimer's disease. Alzheimers Dement. 2018;14:535-562).

It may be important to note that subtle cognitive impairment is a description for cognitive complaints so light that the person barely notices a negative impact on their life. This differs from e.g. mild cognitive impairment.

**Mild cognitive impairment** is defined as slight impairment in cognitive functions with otherwise normal function in the activities of daily living (R.C. Petersen, Mild Cognitive Impairment as a diagnostic entity, J Intern Med 256 (2004), 183-194).

In an example, the determination of the performance index comprises at least one time for the user to perform the cognitive test.

Thus, an overall time to complete the cognitive test can be used to determine the performance index, where for example the test involves presenting a coloured lights to the user and the user has to press a button as soon as possible after a red light is displayed and/or the performance index can be determined from the overall set of user responses for numerous lights shown to the user that takes into account variations in response times for the user and can take into account when the user has pressed the button incorrectly, for example after a green light is shown rather than a red light.

In an example, for a trail making test the input from the user as they trace a trail between targets as they undertake at least some of the calibration Trail Making Test comprises movement of a finger or stylus across a surface of the input/output unit.

In an example, the processing unit is configured to determine a measure of the user's performance in determining a performance index, and wherein the measure of the user's performance is based in part on a time taken for the user to complete the evaluation Trail Making Test.

According to an example, the input/output unit is configured to receive input from the user as they undertake each further cognitive test of the one or more further cognitive tests. The processing unit is configured to determine a further performance index for the user undertaking each of the one or more further cognitive test comprising utilization of the input from the user. The determination of the cognitive assessment can comprise utilization of the further performance index for each of the one or more further cognitive tests.

In this manner, the effort a user expends on a test and their performance can be assessed and it can be determined if the performance is plateauing as the user completes numerous tests and becomes more used to the tests and at the same time the cognitive effort can be assessed to assess if the person has indeed peaked in performance or has become bored with the tests, for example. By comparing cognitive effort and performance it can be determined if the user has not yet reached a plateau and their results may not be particularly valid for assessment, determined if the user has indeed reached a plateau and their results are valid for assessment, and if a person has given up on a task if both cognitive effort and performance rise and then fall for example.

To put this another way, as a person carries out trials of a test (or tests of a battery of tests) their performance starts from a baseline and gradually improves towards a plateau that is representative of their actual cognitive capability with respect to the test(s), and it can then be determined to select those tests at the plateau that are representative for further analysis by a medical professional or test overseer, and not utilise the test results leading up to the plateau. The cognitive effort expended by the person provides further information, where it can be determined if the person has really reached a plateau, or is struggling and/or shows anomalous variability in effort that can indicate that the person has a cognitive impairment.

Thus, regarding the example task of pressing a button when a red dot is presented on a screen the instructions associated with the test is "press the button as quickly as you can only when you see a red dot" and the test results in this case comprise the times between the red dot being presented and the time when the button was pressed for the multiple times the red dot was presented on the screen. The fluctuation in the plurality of trials of the test results then comprises the fluctuations in the times between the red dot being shown and the patient pressing the button, and the index for the patient can then comprise this fluctuation information.

In an example, the determination of the cognitive assessment comprises a determination of a standard deviation associated with the performance index and the further performance indexes.

Thus, for example regarding the example of the patient being asked to press a button when a dot is shown on a screen and a reaction time between the dot being shown and the button is pressed are trials of a test, there could be for example 400 trials. This the enables variations/standard deviation of the user's response time to be determined.

Thus, for example regarding the example of a patient being asked to think of as many words beginning with E, and then P, and then Q, the effort the user is expending determining from bio signals can be determined and the variation in effort can be determined. At the same time the performance of the user can be determined, for example how many words they could think of for a particular letter and/or how many words they could think of for a letter within a set timescale enabling a performance index to be determined across the test that can be used with the cognitive effort index to determine an overall cognitive assessment for the user.

Also, the example of a patient being asked to think of words beginning with E this can also be compartmentalized into bins and the variation across bins analysed. Thus, a 10 minute period could have five 2 minute bins. In the first 2 minutes the patient my think of many correct words, in the second 2 minutes the patient may think of only a few correct words, whilst in the third the patient may only think of incorrect words with an F etc, and this variation and omissions/error rate can be used to determine a performance index and its variation for the user and at the same time the cognitive effort expended by the user in each of these bins, determined from bio signals, is calculated that with the performance index enables an overall cognitive assessment for the user to be determined..

According to an example, the determination of the cognitive assessment for the user comprises a determination of a degree of variability between the performance index and at least one of the further performance indexes.

According to an example, the one or more further cognitive tests are the plurality of further cognitive tests, and the determination of the cognitive assessment for the user comprises a calculation of a curve fitted to the performance index and the further performance indexes for the plurality of further cognitive tests.

Thus, it has been established that model can be fit to the performance improvement of a person as they undertake trials of a test (or tests of a battery of tests) as a performance gradually increases to a plateau. This means that the tests that are at the plateau can be selected for further analysis by medical professional, and the curve itself can be utilised to provide information on the cognitive ability of the person such as through timescale value related to how long it takes the person to reach the plateau and the cognitive effort expended by the person during the taking of the tests provides further information on the cognitive ability of the person. It can also be determined that a person who is not reaching a plateau is bored and not concentrating on the test as there are not expending significant cognitive effort or that they are expending significant cognitive effort but still having trouble completing the tests, that can be indicative of cognitive impairment.

In an example, the determination of the cognitive assessment comprises a determination of a model used to fit the curve to the performance indexes.

In other words, a timescale associated with a model fitted to how a person's performance with respect to trials of a test (or tests of a test battery) gradually increases provides a new and important additional outcome measure of the neurological assessment that can be provided along with the cognitive effort expended by the person to a medical professional/clinician or to an exam overseer. This enables a quantitative assessment to be made regarding how a person's performance is changing to be determined and this along with the cognitive effort expended by the person, and how it too changes, enables an accurate determination of an overall cognitive assessment of the person to be made.

According to an example, the determination of the cognitive assessment for the user comprises a correlation of the cognitive index with the performance index and the one or more further cognitive indexes with the one or more further performance indexes.

According to an example, the determination of the cognitive assessment comprises a comparison of the cognitive effort index with a reference cognitive effort index or a comparison with a cognitive effort threshold.

According to an example, the at least one sensor is configured to acquire at least one bio signal of the user after the user has undertaken the cognitive test. The processing unit is configured to determine a delayed cognitive effort index. The determination of the delayed cognitive effort index can comprise utilization of the at least one bio signal of the user acquired after the user has undertaken the cognitive test. The determination of the cognitive assessment can comprise utilization of the delayed cognitive effort index.

In this manner fatigue can be assessed after the cognitive test or battery of tests, in effect as a proxy for effort given during the test or battery of tests, that this assessment of fatigue can be utilized along with the assessment during the test(s) to provide an overall more accurate cognitive assessment.

In an example, the one or more bio signals comprises one or more of: body balance, gait, sleep patterns, physical activity levels.

In an example, the input/output unit is configured to receive self-report information from the user after the user has undertaken the cognitive test, and wherein the determination of the delayed cognitive effort index comprises utilization of the received self-report information.

In an example, the input/output unit is configured to receive diary characteristics from the user after the user has undertaken the cognitive test, and wherein the determination of the delayed cognitive effort index comprises utilization of the received diary characteristics.

In an example, the input/output unit is configured to receive input from a brief cognitive test from the user after the user has undertaken the cognitive test, and wherein the determination of the delayed cognitive effort index comprises utilization of the received input from the brief cognitive test.

In an example, the brief cognitive test is a Paced Auditory Serial Addition Test PASAT, see e.g. Bryant, D., Chiaravalloti, N. D., & DeLuca, J. (2004). Objective measurement of cognitive fatigue in multiple sclerosis. Rehabilitation psychology, 49(2), 114.).

According to an example, the at least one bio signal comprises one or more of: cardiovascular activity, heart rate, heart rate variability, heart high/low frequency ratio, systolic blood pressure, diastolic blood pressure, electrodermal activity, skin conductance level, nonspecific skin conductance responses, respiration rate, respiration depth, pupil dilation, salivation, electroencephalogram data.

Fig. 3 shows an example of a cognitive assessment method 100. The method 100 comprises

presenting 110 by an input/output unit 20 a cognitive test to a user;
acquiring 120 by at least one sensor 30 at least one bio signal of the user as the user undertakes the cognitive test;
determining 130 by a processing unit 40 a cognitive effort index, wherein the determining the cognitive effort index comprises utilizing the at least one bio signal of the user; and
determining 140 by the processing unit a cognitive assessment, wherein the determining the cognitive assessment comprises utilizing the cognitive effort index.

In an example, the determining the cognitive assessment comprises comparing the cognitive effort index with a reference cognitive effort index or comparison comparing the cognitive effort index with a cognitive effort threshold.

In an example, the method comprises presenting by the input/output unit one or more further cognitive tests to the user, acquiring by the at least one sensor at least one bio signal of the user as the user undertakes each further cognitive test of the one or more further cognitive tests, determining by the processing unit a further cognitive effort index for each of the one or more further cognitive tests comprising utilizing the at least one bio signal of the user for each of the one or more further cognitive tests. The determining the cognitive assessment for the user can then comprise utilizing the further cognitive effort index for each of the one or more further cognitive tests.

In an example, the determining the cognitive assessment for the user comprises determining a degree of variability between the cognitive effort index and at least one of the further cognitive effort indexes.

In an example, the one or more further cognitive tests are a plurality of further cognitive tests. The determining the cognitive assessment for the user can comprise calculating a curve fitted to the cognitive effort index and the further cognitive effort indexes for the plurality of further cognitive tests.

In an example, the method comprises receiving by the input/output unit input from the user as they undertake the cognitive test, determining by the processing unit a performance index for the user undertaking the cognitive test comprising utilizing the input from the user. The determining the cognitive assessment can comprise utilizing the performance index.

In an example, the method comprises receiving by the input/output unit input from the user as they undertake each further cognitive test of the one or more further cognitive tests, determining by the processing unit a further performance index for the user undertaking each of the one or more further cognitive test comprising utilizing the input from the user. The determining the cognitive assessment can then comprise utilizing the further performance index for each of the one or more further cognitive tests.

In an example, the determining the cognitive assessment for the user comprises determining a degree of variability between the performance index and at least one of the further performance indexes.

In an example, the one or more further cognitive tests are the plurality of further cognitive tests. The determining the cognitive assessment for the user can comprise calculating a curve fitted to the performance index and the further performance indexes for the plurality of further cognitive tests.

In an example, the determining the cognitive assessment for the user comprises correlating the cognitive index with the performance index and the one or more further cognitive indexes with the one or more further performance indexes.

In an example, the determining the cognitive assessment comprises comparing the cognitive effort index with a reference cognitive effort index or comparing the cognitive effort index with a cognitive effort threshold.

In an example, the method comprises acquiring by the at least one sensor at least one bio signal of the user after the user has undertaken the cognitive test, determining by the processing unit a delayed cognitive effort index comprising utilizing the at least one bio signal of the user acquired after the user has undertaken the cognitive test. The determining the cognitive assessment can then comprise utilizing the delayed cognitive effort index.

In an example, the at least one bio signal comprises one or more of: cardiovascular activity, heart rate, heart rate variability, heart high/low frequency ratio, systolic blood pressure, diastolic blood pressure, electrodermal activity, skin conductance level, nonspecific skin conductance responses, respiration rate, respiration depth, pupil dilation, salivation, electroencephalogram data.

The cognitive assessment apparatus and the cognitive assessment method are now described in further specific detail, where reference is made to Figs, 4-7.

Typically practice effects are viewed as a source of bias in repeated neuropsychological assessments. In other words, the patient's performance can change as they repeat tests. Importantly, it was also realized by the inventors that the effort expended by a patient as they conduct trials of a test and/or tests of a battery of tests can also change as they carry out the trials and/or carry out the tests of the battery of tests.

It was realized by the inventors that how the effort expended by the patient changes and how the performance changes can provide additional information about the person's cognitive capabilities, in order to provide a cognitive assessment of the patient. This led to the development of the new cognitive assessment apparatus and the cognitive assessment method described here.

The following discussion on quantitative monitoring centers on monitoring the performance of the patient, but equivalent quantitative monitoring can be carried out with respect to the effort expended by the patient, monitored through acquired bio signals.

Thus, if a person takes longer to reach a performance and/or effort plateau this may be an early indication of a change in cognitive functioning. In disorders where learning is compromised, such as Alzheimer's disease (AD) or mild cognitive impairment (MCI), attenuated practice effects can be expected. In general or on specific tasks - e.g., lower practice effects on episodic memory tasks have been related to AD dementia (see for example: Jutten, R. J., Grandoit, E., Foldi, N. S., Sikkes, S. A., Jones, R. N., Choi, S. E., ... & Rabin, L. A. (2020). Lower practice effects as a marker of cognitive performance and dementia risk: a literature review. Alzheimer's & Dementia: Diagnosis, Assessment & Disease Monitoring, 12(1), e12055). In other words; variability in practice effects can be used to define cases or predict longitudinal outcomes.

Thus, in the new technique described here providing a comparison of learning curves to a normalized average, between longitudinal assessments, or between cognitive domains can therefore provide important additional information on the change in a person's neuropsychology. Therefore, a method to detect, quantify and compare these practice effects was developed to enable these beneficial effects.

Typically, each neuropsychological assessment (or test battery) consists of number of neuropsychological tests (e.g. RAVLT, TMT, etc.). In each test the person is presented with a number of items and a logical sequence of (presenting and processing responses to) items is called a trial. Some tests can have only one trial. An assumption was made in the new development that after each trial the next one will be executed faster or with less errors. It was then taken that this general learning process will continue until hardly any improvement is observable anymore: the performance plateau is reached. It was then determined in the new technique that this general learning process can be described with the following model formula:

$$SSasymp(t) = Asym + (Perf0 - Asym) * \exp(-t/tau)$$

Where:

*t*  = a numeric vector that represents trial number 1, 2,..

*Asym*  = a numeric parameter representing the upper asymptotic value of the model

*Perf0*  = a numeric parameter representing the performance at t=0 (i.e., before training)

*tau*  = a numeric parameter representing the learning constant

The relative error between succeeding trials can be defined by the following formula:

$$rel.\,error[i] = 2 * \frac{Perf[i] - Perf[i-1]}{Perf[i] + Perf[i-1]}$$

Applying these two formulas to trial data the performance plateau and the time to reach it can be estimated without executing a very large number of training sessions. Equivalent formulas can be constructed with respect to the effort expended by the patient

For people with a small *tau* - reaching their performance plateau after only a few trials - the relative error between two succeeding training trails can be sufficient to determine their plateau. That is if the difference in performance and/or effort on trial *i* vs trial *i-1* is smaller than for instance 5% the person has reached a plateau in performance and/or effort. There can be multiple explanations for a small tau including pre (at-home) training, having done a similar assessment recently, or high cognitive function.

For people with a larger *tau* - reaching their performance and/or effort plateau after >3 trials - a plateau in performance and/or effort can be estimated by fitting a non-linear model to the performance data (see for example: Venables, W. N., & Ripley, B. D. (2003). Modern Applied Statistics With S. (J. Chambers, W. Eddy, W. Härdle, S. Sheather, & L. Tierney, Eds.), Technometrics (Fourth Edi, Vol. 45). New York, NY: Springer Science+Business Media). Considering the 3 parameters of the model formula equation above, data of at least 4 trails are needed. Each additional trial will make the estimate more reliable.

Thus, in a specific embodiment of the new technique the first step is assessing the patient's variability between consecutive trials for <3 trials. If the variability between these trials is smaller than for instance 5% it can be safely assumed that the patient is on their performance and/or effort plateau. Thus, fitting the performance data and/or effort to a curve is not needed. However, if the variability in the first 3 trials is larger than for instance 5% more trials are needed, and the patient's performance and/or effort should be fitted to a general learning curve to estimate plateau. Using these two formulas the performance and/or effort plateau can be estimated regardless of any pretraining of the patient. It is to be noted that a threshold of 5% can be set initially, as this is a common threshold used in statistics, and the clinician can re-evaluate this threshold at her/his own discretion and increase it or reduce it as necessary.

It is to be noted that by fitting the performance and/or effort data to a curve the plateau can be predicted before it is reached. This feature can be especially valuable for patients that need a very large number of trials to reach their plateau. In this case a reliable estimated of learning ability and performance and/or effort can be provided without the need to complete all trials. Thus, a large amount of time is saved. Also, by fitting the performance and/or effort data to a curve, differences in the learning curve between patients or for the same patient over time can be detected, where the difference in the curves can be represented by differences in tau. This difference in tau can be interpreted using contextual knowledge such as time between testing, degeneration on MRI niveau, age, and task complexity.

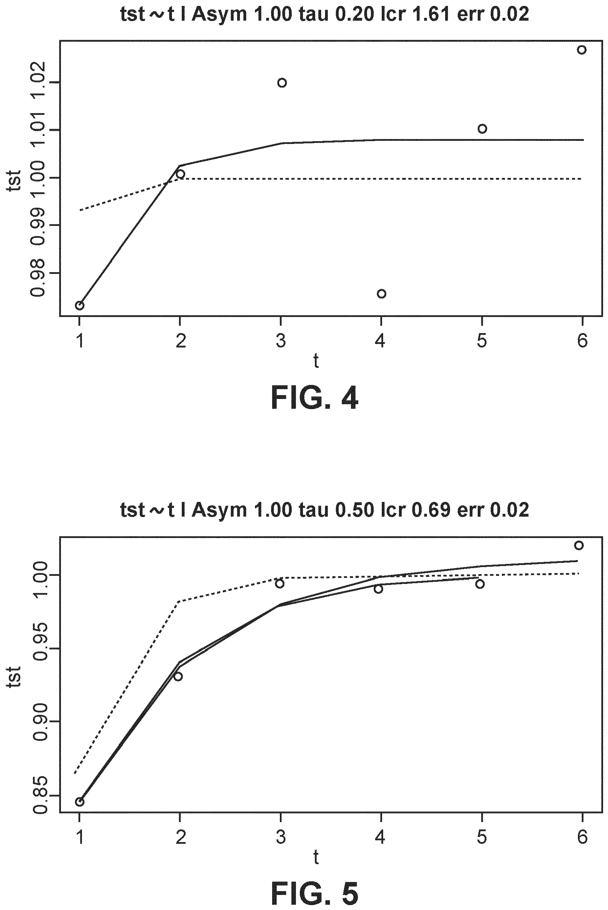

Figs. 4-7 then show some illustrative examples for performance data, showing cases that differ in terms of how fast the person learns *(tau:* that is the time to reach the plateau). In the figures *tst* stands for performance and t for the number of trials.

In Fig. 4, this person reached a plateau in performance after 2 trials because the relative error between trial 1 and 2 is smaller than 5% (2.8%).

In Fig. 5, this person reached a plateau in performance after 4 trials, because the relative error between trial 3 and 4 is dropped below 5% (0.39%). Because the person already executed 4 trials a non-linear fit with the learning model can be performed to give an estimations and error margin of the asymptotic value ($p < 0.05$), *tau* ($p > 0.1$) and response at time 0 ($p > 0.1$).

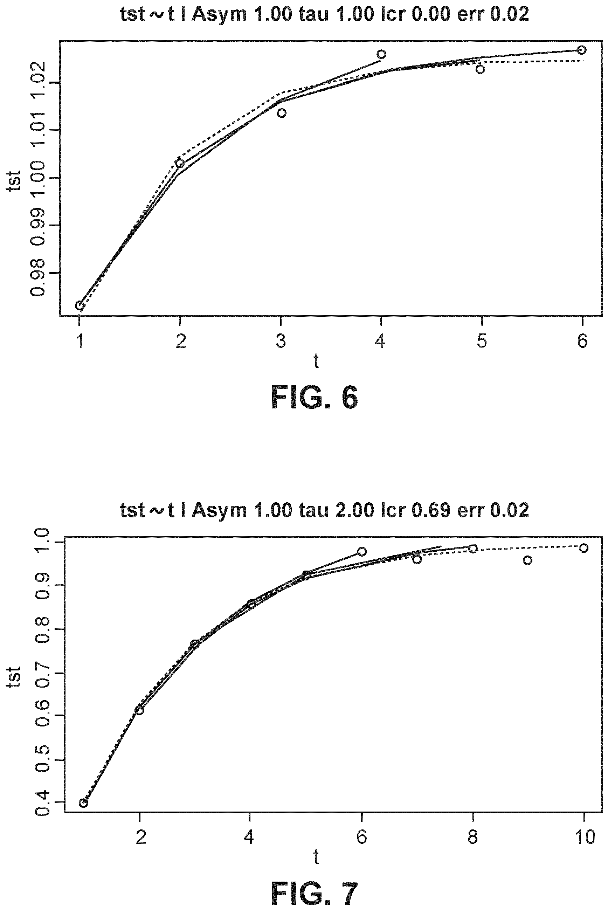

In Fig. 6, this person reached a plateau in performance after 5 g trials, because the relative performance dropped below 5% between trial 4 and 5 (2.0%). The non-linear model can use now 5 data points to give more accurate parameter estimations.

In Fig. 7, this person reached a plateau in performance after 7 trials, because the relative performance dropped below 5% between trial 6 and 7 (1.7%). The non-linear model can use now 7 data points to give more accurate parameter estimations.

As discussed above, the new technique in a specific embodiment involves 1. fitting performance and/or effort data to a general learning curve, and/or 2. considering the relative error between trials as an indicator of performance and/or effort plateau.

Here, performance and/or effort can be fitted to a general learning curve, one neuropsychological assessment can consist of one or multiple tests, with each test consisting of multiple trials, and here a longitudinal assessment is comparing assessments over time.

The inventors realized however that mTBI patients (and patients with another neurological condition that can cause subtle cognitive impairment, e.g. MS or stroke) may perform a neuropsychological assessment within the norm, but it may cost them a lot of effort (more than the norm) to complete the assessment. As a result, they also need more time to recover from the test afterwards. In other words, assessment of performance of a test may not be able to determine if a patient has a subtle cognitive impairment, but assessment of the effort expended by the patient can be used to determine if the patient has a subtle cognitive impairment. Then, however the information on the performance taken along with the information of effort can be used to provide further in formation on the subtle cognitive impairment as determined from the monitoring of effort expended. Thus, cognitive effort is considered to be an additional novel outcome measure of a cognitive test, providing the benefit of increased test sensitivity.

To put this another way, the inventors realised that standard (paper-pencil) cognitive tests are not sensitive enough to pick up subtle cognitive impairment. However, the measurement of effort during a cognitive test provides an additional outcome measure that can be used to pick up subtle cognitive impairment. Cognitive effort is not a clearly defined concept, and it may be related to cognitive fatigue, cognitive/mental load, cognitive stress, and similar concepts. Cognitive effort is currently not measured in a neuropsychological assessment, and the inventors measured this through acquiring bio signals unobtrusively using wearable or vital eye technology. The inventors in developing this new technique took into account that learning, mental workload, and effort are reflected in peripheral physiological arousal (see: Charles, R. L., & Nixon, J. (2019). Measuring mental workload using physiological measures: A systematic review. Applied Ergonomics, 74, 221-232, https://doi.org/10.1016/JAPERGO.2018.08.028; Fairclough, S. H., Venables, L., & Tattersall, A. (2005). The influence of task demand and learning on the psychophysiological response. International Journal of Psychophysiology, 56(2), 171-184; and Miyake, S., Yamada, S., Shoji, T., Takae, Y., Kuge, N., & Yamamura, T. (2009). Physiological responses to workload change. A test/retest examination. Applied Ergonomics, 40(6), 987 -996), which can be measured through bio signals.

The following helps to show how performance may not be able to indicate an impairment, whilst effort can be used to indicate an impairment. For illustrative purposes the following relates to a comparison of running for two people, but the equivalent can apply to two people who undertake the same cognitive test, and who have equivalent performance. Thus, to illustrate, with the running comparison; two people may run 1 km at the same speed. But one person has a heart rate of 100 bpm and the other a heart rate of 190 bpm. Without physiological insight, you do not know that the second person had to put in more effort and needs longer recovery. This insight has been used by the inventors with respect to monitoring effort via bio signals of patients undertaking cognitive tests in order to measure effort from which subtle cognitive impairment can be indicated where the performance alone as such from the test cannot.

In developing the new technique, the inventors took into account the following work and information. Psychological processes correspond with the activation of brain areas, such as the prefrontal cortex, limbic system, or thalamus (Gazzaniga et al. 2009; Posner et al. 2005). In turn, these brain areas can further activate the peripheral nervous system (Fairclough et al. 2014; Jänig 2003; Kreibig 2010; Picard et al. 2001; Thayer and Lane 2009). The autonomic nervous system innervates bodily processes via its sympathetic and parasympathetic branches. Via the sympathetic branch, the body is activated and prepared for action whereas the parasympathetic branch is responsible for relaxation (Jänig 2003). Sympathetic arousal is defined as any change in the relevant parameters (e.g. increase in heart rate (HR), skin conductance level (SCL), number of skin conductance responses (SCRs), and decrease in heart rate variability (HRV)). The interplay between the two branches determines the activity in the various physiological subsystems (Cacioppo et al. 2007; Jänig 2003), such as the cardiovascular, electrodermal, respiratory, and facial muscle systems (fig. 2). As such these systems are known to reflect different parts of emotional (Jänig 2003; Kreibig 2010; Picard et al. 2001) and cognitive processes (Boucsein 2012; Fairclough and Mulder 2011).

The inventors then consider the following examples of measurable features of sympathetic activation:

Cardiovascular activity: heart rate, heart rate variability, high/low-frequency ratio, systolic blood pressure, diastolic blood pressure, blood flow
Electrodermal activity: skin conductance level, (nonspecific) skin conductance responses Respiration rate and depth
Pupil dilation
Salivation
Brain activity via Electroencephalogram (EEG)

Thus, the new technique or approach relates to measuring cognitive effort during a neuropsychological assessment by means of physiological assessment. This approach provide additional information that can inform clinicians' diagnosis of cognitive impairment in people with subtle cognitive impairment (e.g. mTBI, MS, stroke). It comprises the following elements:

An interface to present a cognitive test or test battery
A means to capture at least one bio signal
A processing unit (algorithm): collecting the cognitive test stimuli and bio signal input and estimating cognitive effort
An interface to measure the effort estimate as an
additional outcome measure

The following relates to a number of different embodiments/variations of the new technique.

**Increased physiological arousal relates to more effort.**

Here cognitive effort is measured unobtrusively during the neuropsychological assessment using wearable or vital eye technology that measures some kind of bio signal, e.g. skin conductance, heart rate variability, blood flow, EEG. From these bio signals, effort can be detected by using for instance pre-set or relative thresholds. One example of a pre-set threshold is the use of heart rate variability (HRV). For example high frequency HRV between 0.15-0.4 Hz relates to high effort. Another example of a relative threshold is the use of skin conductance. More effort for a patient to conduct a test means increased skin conductance with respect to a baseline measurement (measured by known techniques that make use of conventional metrics). In addition, heart rate can also be measured which also relates to effort as well as body motion of a patient (via accelerometery). A combination of bio signals (e.g., skin conductance, heart rate, and motion detection) can be even more informative.

**Variability in physiology**

The variability in cognitive effort can be assessed between tests within a test battery/neuropsychological assessment. Patients with subtle cognitive impairment spend more cognitive effort on certain cognitive tests that require the recruitment of cognitive functions that are impaired due to the underlying damage or disease. Cognitive tests that measure cognitive functions that are less impaired may require relatively less cognitive effort. In other words, for people with subtle cognitive impairment, more variability is detected in cognitive effort between different cognitive tests of a neuropsychological assessment compared to healthy people. Thus, variability in effort (as measured by physiological arousal) can indicate the presence of subtle cognitive impairment that would otherwise not be detected.

**Physiology and performance for performance plateau detection**

Both physiological arousal (effort) and performance can be taken into account and used to estimate a plateau (saturation) in learning. Physiological measures can be used to check whether the person's performance scores are at a plateau because he or she is bored or tired (no or low sympathetic arousal and expectedly a low performance) or because they are at the peak of their performance (moderate or high sympathetic arousal and expectedly high performance).

Low level of effort: Given both performance and physiological arousal are low, we can assume a performance plateau is not reached within the time given. Expectedly this yields unreliable test results.

Healthy level of effort: Both performance and sympathetic arousal make a significant increase from baseline/start and then flatline at a reached peak. Thus, it can be assumed that a performance plateau is reached.

High level of effort: Both performance and sympathetic arousal make a significant increase from baseline/start, but then decline again. It can be assumed that the person has given up on the task.

In a closely related embodiment, this approach can be used to adapt task difficulty to a person's capabilities. Based on current load, the processor continuously adapts the difficulty of the training towards cognitive redline: Making the training more difficult if the patient appears to be underloaded, or less difficult if the patient appears to be overloaded. For example, certain characteristics of the Stroop task can be adapted to modify the difficulty. Exemplary characteristics are the percentage congruent versus incongruent trials, the number of colours used in the task, adapting the speed of presentation of stimuli. The difficulty can be decreased by presenting a higher percentage of congruent word-colour trials, or by decreasing the number of colours used in the task, or by increasing the presentation duration of stimuli. Expectedly, adapting the training difficulty to the patient ensures that the performance plateau is reached faster than in a non-adaptive setting. Optionally, this is done within a set timeframe; if the patient has not reached the plateau within e.g. 5 minutes, lower the training difficulty to the ability of the patient in order to speed up the process. Optionally, a display unit shows the measured and processed signals to the assessor, indicates when a performance plateau is reached or stops the training.

## Measurement of cognitive fatigue during NPA.

Cognitive fatigue is a symptom of many neurological diseases including mTBI, MS and stroke. The new technique can be used as a method to objectively assess cognitive fatigue in patients with other neurological diseases. One example is stroke. A stroke patient may have visual impairment as a result of the stroke which can influence the patient's performance of a cognitive test. In a subset of patients, a stroke can cause visual neglect which means that the patient does not pay attention to visual targets in one half of the visual field (left or right). Now assume that the patient scored well (during another cognitive test) on using visual targets throughout the visual field. This then means that the patient does not suffer from visual neglect. This, in turn, means that increased effort on e.g., a memory test that includes targets throughout the visual field cannot be caused by visual neglect, and must be caused by increased effort. In this case, the bio signal (eye gaze in this case) can be interpreted as being associated to the cognitive impairment and not to a visual neglect impairment. Another example concerns MS, which would be similar to the visual impairment example, but then for motor impairment.

## Recovery time as proxy for effort given during NPA

Cognitive effort can also be measured after the neuropsychological assessment, using home monitoring technology. Cognitive effort - or the cognitive fatigue/exhaustion thereafter - may often only become apparent to patients with subtle cognitive impairment in the hours after they have performed the neuropsychological assessment. Cognitive fatigue/exhaustion can therefore be measured after the neuropsychological assessment. This can be done using subjective reporting in a home monitoring application such as self-report measures as well as brief cognitive tests that specifically measure aspects of cognitive fatigue such as the ability to sustain attention, e.g. the Paced Auditory Serial Addition Test (PASAT, see e.g. Bryant et al., 2004). Alternatively or additionally, with for example a smartwatch and/or a diary characteristics like physical activity levels, gait, sleep patterns, resting heart rate, body balance, subjective states, in the days before versus the days after the NPA can be compared. This can give an additional objective estimation of cognitive fatigue/exhaustion.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1.  A cognitive assessment apparatus (10), comprising:

    - an input/output unit (20);
    - at least one sensor (30); and
    - a processing unit (40);

      wherein the input/output unit is configured to present a cognitive test to a user;
      wherein the at least one sensor is configured to acquire at least one bio signal of the user as the user undertakes the cognitive test;
      wherein the processing unit is configured to determine a cognitive effort index, wherein the determination of the cognitive effort index comprises utilization of the at least one bio signal of the user; and
      wherein the processing unit is configured to determine a cognitive assessment, wherein the determination of the cognitive assessment comprises utilization of the cognitive effort index.

2.  Apparatus according to claim 1, wherein determination of the cognitive assessment comprises a comparison of the cognitive effort index with a reference cognitive effort index or a comparison with a cognitive effort threshold.

3.  Apparatus according to any of claims 1-2, wherein the input/output unit is configured to present one or more further cognitive tests to the user; wherein the at least one sensor is configured to acquire at least one bio signal of the user as the user undertakes each further cognitive test of the one or more further cognitive tests; wherein the processing unit is configured to determine a further cognitive effort index for each of the one or more further cognitive tests, wherein the determination of the further cognitive effort index comprises utilization of the at least one bio signal of the user for each of the one or more further cognitive tests; and wherein the determination of the cognitive assessment for the user comprises utilization of the further cognitive effort index for each of the one or more further cognitive tests.

4.  Apparatus according to claim 3, wherein the determination of the cognitive assessment for the user comprises a determination of a degree of variability between the cognitive effort index and at least one of the further cognitive effort indexes.

5.  Apparatus according to any of claims 3-4, wherein the one or more further cognitive tests are a plurality of further cognitive tests, and wherein the determination of the cognitive assessment for the user comprises a calculation of a curve fitted to the cognitive effort index and the further cognitive effort indexes for the plurality of further cognitive tests.

6.  Apparatus according to any of claims 1-5, wherein the input/output unit is configured to receive input from the user as they undertake the cognitive test, wherein the processing unit is configured to determine a performance index for the user undertaking the cognitive test comprising utilization of the input from the user, and wherein the determination of the cognitive assessment comprises utilization of the performance index.

7.  Apparatus according to claim 6 when dependent upon any of claims 3-5, wherein the input/output unit is configured to receive input from the user as they undertake each further cognitive test of the one or more further cognitive tests; wherein the processing unit is configured to determine a further performance index for the user undertaking each of the one or more further cognitive test comprising utilization of the input from the user; and wherein the determination of the cognitive assessment comprises utilization of the further performance index for each of the one or more further cognitive tests.

8. Apparatus according to claim 7, wherein the determination of the cognitive assessment for the user comprises a determination of a degree of variability between the performance index and at least one of the further performance indexes.

9. Apparatus according to any of claims 7-8, wherein the one or more further cognitive tests are the plurality of further cognitive tests, and wherein the determination of the cognitive assessment for the user comprises a calculation of a curve fitted to the performance index and the further performance indexes for the plurality of further cognitive tests.

10. Apparatus according to any of claims 7-8, wherein the determination of the cognitive assessment for the user comprises a correlation of the cognitive index with the performance index and the one or more further cognitive indexes with the one or more further performance indexes.

11. Apparatus according to any of claims 1-10, wherein determination of the cognitive assessment comprises a comparison of the cognitive effort index with a reference cognitive effort index or a comparison with a cognitive effort threshold.

12. Apparatus according to any of claims 1-11, wherein the at least one sensor is configured to acquire at least one bio signal of the user after the user has undertaken the cognitive test; wherein the processing unit is configured to determine a delayed cognitive effort index, wherein the determination of the delayed cognitive effort index comprises utilization of the at least one bio signal of the user acquired after the user has undertaken the cognitive test; and wherein the determination of the cognitive assessment comprises utilization of the delayed cognitive effort index.

13. Apparatus according to any of claims 1-12, wherein the at least one bio signal comprises one or more of: cardiovascular activity, heart rate, heart rate variability, heart high/low frequency ratio, systolic blood pressure, diastolic blood pressure, electrodermal activity, skin conductance level, nonspecific skin conductance responses, respiration rate, respiration depth, pupil dilation, salivation, electroencephalogram data.

14. A cognitive assessment method (100), comprising:

presenting (110) by an input/output unit (20) a cognitive test to a user;
acquiring (120) by at least one sensor (30) at least one bio signal of the user as the user undertakes the cognitive test;
determining (130) by a processing unit (40) a cognitive effort index, wherein the determining the cognitive effort index comprises utilizing the at least one bio signal of the user; and
determining (140) by the processing unit a cognitive assessment, wherein the determining the cognitive assessment comprises utilizing the cognitive effort index.

15. A computer program element for controlling an apparatus according to any of claims 1-3 which when executed by a processor is configured to carry out the method of claim 14.

FIG. 1

20    40    30    10

FIG. 2

100

110

120

130

140

FIG. 3

tst ~ t I Asym 1.00 tau 0.20 lcr 1.61 err 0.02

FIG. 4

tst ~ t I Asym 1.00 tau 0.50 lcr 0.69 err 0.02

FIG. 5

tst ~ t I Asym 1.00 tau 1.00 lcr 0.00 err 0.02

FIG. 6

tst ~ t I Asym 1.00 tau 2.00 lcr 0.69 err 0.02

FIG. 7

Europäisches
Patentamt
European
Patent Office
Office européen
des brevets

## EUROPEAN SEARCH REPORT

Application Number

EP 22 20 3205

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/086729 A1 (BRUNO JEFF SCOTT [US]) 30 March 2017 (2017-03-30) * paragraph [0013] - paragraph [0014] * * paragraph [0030] - paragraph [0048] * * paragraph [0055] - paragraph [0056] * * paragraph [0064] - paragraph [0069] * * paragraph [0075] - paragraph [0131] * * figures * | 1-15 | INV. A61B5/00 |
| X | US 2014/171756 A1 (WALDORF RONALD A [US] ET AL) 19 June 2014 (2014-06-19) * abstract * * paragraph [0002] * * paragraph [0007] * * paragraph [0020] - paragraph [0021] * * paragraph [0028] - paragraph [0038] * * paragraph [0048] * * paragraph [0059] - paragraph [0060] * * paragraph [0067] * * paragraph [0077] - paragraph [0093] * * figures 1,2,4,10A-B * | 1-4,11, 13-15 | |
| A | VILLANI VALERIA ET AL: "Wearable Devices for the Assessment of Cognitive Effort for Human-Robot Interaction", IEEE SENSORS JOURNAL, IEEE, USA, vol. 20, no. 21, 10 June 2020 (2020-06-10) , pages 13047-13056, XP011812372, ISSN: 1530-437X, DOI: 10.1109/JSEN.2020.3001635 [retrieved on 2020-10-01] * the whole document * | 1,3,6,7, 13-15 | TECHNICAL FIELDS SEARCHED (IPC) A61B |

-/--

The present search report has been drawn up for all claims

| Place of search | Date of completion of the search | Examiner |
|---|---|---|
| The Hague | 22 February 2023 | Van der Haegen, D |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches Patentamt
European Patent Office
Office européen des brevets

# EUROPEAN SEARCH REPORT

Application Number

EP 22 20 3205

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARSHALL SANDRA P: "The index of cognitive activity: measuring cognitive workload", PROCEEDINGS OF THE 2002 IEEE 7TH. CONFERENCE ON HUMAN FACTORS AND POWER PLANTS. SCOTTSDALE, AZ, SEPT. 15 – 19, 2002; [IEEE CONFERENCE ON HUMAN FACTORS AND POWER PLANTS], NEW YORK, NY : IEEE, US, vol. CONF. 7, 15 September 2002 (2002-09-15), pages 7_5-7_9, XP010613086, ISBN: 978-0-7803-7450-8 * the whole document * | 1,13-15 | |
| A | GREEN M W ET AL: "Impairment of cognitive performance associated with dieting and high levels of dietary restraint", PHYSIOLOGY AND BEHAVIOR, ELSEVIER SCIENCE LTD., OXFORD, GB, vol. 55, no. 3, 1 March 1994 (1994-03-01), pages 447-452, XP024317659, ISSN: 0031-9384, DOI: 10.1016/0031-9384(94)90099-X [retrieved on 1994-03-01] * abstract * * page 448 – page 450 * * table 3 * | 12 | TECHNICAL FIELDS SEARCHED (IPC) |

The present search report has been drawn up for all claims

| Place of search | Date of completion of the search | Examiner |
|---|---|---|
| The Hague | 22 February 2023 | Van der Haegen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 3205

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2023

| Patent document cited in search report | | Publication date | Patent family member(s) | | Publication date |
|---|---|---|---|---|---|
| US 2017086729 | A1 | 30-03-2017 | US | 2017086729 A1 | 30-03-2017 |
| | | | US | 2020265942 A1 | 20-08-2020 |
| US 2014171756 | A1 | 19-06-2014 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KARLSEN, R. H. ; SAKSVIK, S. B. ; STENBERG, J. ; LUNDERVOLD, A. J. ; OLSEN, A. ; RAUTIO, I. ; SKANDSEN, T.** Examining the Subacute Effects of Mild Traumatic Brain Injury Using a Traditional and Computerized Neuropsychological Test Battery. *Journal of Neurotrauma,* 2021, vol. 38 (1), 74-85
- **VERMEENT, S. ; DE OLIVEIRA, M. ; SCHMAND, B.** *Which cognitive processes are involved in the Trail Making Test? Towards a more precise neuropsychological assessment using digital tablet-based technology,* 2020
- **BOWIE, C. R. ; HARVEY, P. D.** Administration and interpretation of the Trail Making Test. *Nature protocols,* 2006, vol. 1 (5), 2277-2281
- **STUSS, D.T.** Cognitive Impairment. *Encyclopedia of the Neurological Sciences,* 2003, 737-740
- **JACK CR JR ; BENNETT DA ; BLENNOW K et al.** NIA-AA research framework: toward a biological definition of Alzheimer's disease. *Alzheimers Dement.,* 2018, vol. 14, 535-562
- **R.C. PETERSEN.** Mild Cognitive Impairment as a diagnostic entity. *J Intern Med,* 2004, vol. 256, 183-194
- **BRYANT, D. ; CHIARAVALLOTI, N. D. ; DELUCA, J.** Objective measurement of cognitive fatigue in multiple sclerosis. *Rehabilitation psychology,* 2004, vol. 49 (2), 114
- **JUTTEN, R. J. ; GRANDOIT, E. ; FOLDI, N. S. ; SIKKES, S. A. ; JONES, R. N. ; CHOI, S. E. ; RABIN, L. A.** Lower practice effects as a marker of cognitive performance and dementia risk: a literature review. *Alzheimer's & Dementia: Diagnosis, Assessment & Disease Monitoring,* 2020, vol. 12 (1), e12055
- Modern Applied Statistics With S. **VENABLES, W. N. ; RIPLEY, B. D.** Technometrics. Springer Science+Business Media, 2003, vol. 45
- **CHARLES, R. L. ; NIXON, J.** Measuring mental workload using physiological measures: A systematic review. *Applied Ergonomics,* 2019, vol. 74, 221-232, https://doi.org/10.1016/JAPERGO.2018.08.028
- **FAIRCLOUGH, S. H. ; VENABLES, L. ; TATTERSALL, A.** The influence of task demand and learning on the psychophysiological response. *International Journal of Psychophysiology,* 2005, vol. 56 (2), 171-184
- **MIYAKE, S. ; YAMADA, S. ; SHOJI, T. ; TAKAE, Y. ; KUGE, N. ; YAMAMURA, T.** Physiological responses to workload change. A test/retest examination. *Applied Ergonomics,* 2009, vol. 40 (6), 987-996